Europäisches Patentamt

European Patent Office

Office européen des brevets

(19)

(11) Veröffentlichungsnummer : **0 119 389
B1**

(12) **EUROPÄISCHE PATENTSCHRIFT**

(45) Veröffentlichungstag der Patentschrift :
29.07.87

(21) Anmeldenummer : 84100274.4

(22) Anmeldetag : 12.01.84

(51) Int. Cl.⁴ : **C 07 B 37/12**, C 07 C 13/43,
C 07 C 7/20

(54) Verfahren zum Verhindern der Bildung von polymeren Nebenprodukten bei der Herstellung von 5-Vinyl-norbornen (2).

(30) Priorität : 03.03.83 DE 3307486

(43) Veröffentlichungstag der Anmeldung :
26.09.84 Patentblatt 84/39

(45) Bekanntmachung des Hinweises auf die Patenterteilung : 29.07.87 Patentblatt 87/31

(84) Benannte Vertragsstaaten :
BE CH DE FR GB IT LI NL

(56) Entgegenhaltungen :
EP-A- 0 062 626
DE-A- 2 051 548
DE-A- 2 326 836
DE-C- 2 232 300
Beilsteins Handbuch der organischen Chemie, 1 Ergänzungswerk, Bd. 11u.12, S. 294

(73) Patentinhaber : **BUNAWERKE HÜLS GMBH
Postfach 1320
D-4370 Marl 1 (DE)**

(72) Erfinder : **Donike, Wilhelm, Dr.
Uerdinger Strasse 3
D-4370 Marl (DE)**
Erfinder : **Ulrich, Helmut
Krampenfeld 48
D-4270 Dorsten (DE)**
Erfinder : **Thiemer, Heinz
Ostpreussenstrasse 16
D-4370 Marl (DE)**
Erfinder : **Pieper, Josefa
Heinrich-Rumpf-Strasse 8
D-4358 Haltern (DE)**

**0 119 389**

**Beschreibung**

Aus dem Stand der Technik ist ein Verfahren zum Verhindern der Bildung von polymeren Nebenprodukten bei der Herstellung von 5-Vinyl-bicyclo-[2.2.1]-hepten-(2), das ist 5-Vinylnorbornen-(2), durch Diels-Alder-Reaktion von 1,3-Butadien (Dienkomponente) mit Cyclopentadien und/oder Dicyclopentadien (dienophile Komponente) bekannt.

Das Verfahren weist die folgenden Merkmale auf : die Reaktionskomponenten und eine wirksame Menge eines Polymerisationsinhibitors werden unter (nahezu) vollständigem Ausschluß von Sauerstoff zusammengegeben ; das erhaltene Gemisch wird im Falle der Abwesenheit von Dicyclopentadien auf 140 bis 220 °C oder im Falle der Gegenwart von Dicyclopentadien auf 160 bis 220 °C erhitzt.

In den DE-PSS 22 32 300 und 23 26 836 werden N-Nitrosamine, insbesondere N-Nitrosodiphenylamin, als Polymerisationsinhibitor vorgeschlagen.

In der DE-PS 25 10 481 werden N,N'-disubstituierte p-Phenylendiamine als Polymerisationsinhibitor vorgeschlagen.

Die Wirkungsweise der Polymerisationsinhibitoren in den geschilderten Verfahren des Standes der Technik ist nicht im einzelnen geklärt. Es ist lediglich bekannt, daß N-Nitrosodiphenylamin in der Hitze NO abspaltet (Beilstein, 1. Ergänzungswerk, Band XI/XII, (Berlin 1933), Seite 294, letzter Absatz ; EP-OS 0 062 626, Seite 3).

Die Aufgabe der Erfindung ist es, ein wirtschaftlicheres Verfahren zum Verhindern der Bildung von polymeren Nebenprodukten bei der Herstellung von 5-Vinylnorbornen-(2) bereitzustellen.

Die Aufgabe wurde in überraschender Weise durch die Bereitstellung eines Verfahrens gelöst, das zusätzlich zu den oben angegebenen Merkmalen das folgende Merkmal aufweist : der Polymerisationsinhibitor ist Stickstoffmonoxid (NO) und/oder Distickstofftrioxid ($N_2O_3$).

Das Verfahren kann in Gegenwart eines inerten, organischen Lösemittels durchgeführt werden.

Besonders vorteilhafte Lösungen der Aufgabe wurden durch die Bereitstellung des Verfahrens gemäß den Patentansprüchen 2 bis 6 erzielt.

Das Verfahren gemäß den Patentansprüchen 5 und 6 gestattet die Herstellung von 5-Vinylnorbornen-(2) mit hohen Ausbeuten, bezogen auf die umgesetzten Komponenten, d. h. mit hohen Selektivitäten, da nicht nur entsprechend der Aufgabe die Bildung von polymeren Nebenprodukten verhindert, sondert darüber hinaus die Bildung von oligomeren und niedermolekularen Nebenprodukten, wie beispielsweise 4-Vinylcyclohexen-(1) und Tetrahydroinden, eingeschränkt wird.

Die hohen Selektivitäten resultieren u. a. aus der Tatsache, daß in den angegebenen, engen Temperatur- und Druckbereichen stets eine Mischphase vorliegt, worunter die Existenz einer gasförmigen neben einer flüssigen Phase verstanden wird.

Die Abhängigkeit der Selektivitäten vom Molverhältnis und von den Umsätzen der Komponenten ist dem Fachmann grundsätzlich bekannt (DE-PS 21 61 215 und DD-PS 94 175).

Das Verfahren der Erfindung erweist sich als besonders vorteilhaft, da es kontinuierlich während einer langen Zeit durchgeführt werden kann, ohne daß unerwünschtes Polymerisat auftritt.

Die Wirkungsweise des Polymerisationsinhibitors ist nicht im einzelnen geklärt. Er ist offenbar in der Lage, sowohl die Polymerisation von 1,3-Butadien und (Di)cyclopentadien als auch die Polymerisation von 5-Vinylnorbornen-(2) zu verhindern (siehe Beispiele).

5-Vinylnorbornen-(2) ist von technischer Bedeutung. Es kann in bekannter Weise zu 5-Ethylidennorbornen-(2) isomerisiert werden. Dieses dient als Dien mit nicht-konjugierten, olefinischen Doppelbindungen unterschiedlicher Reaktivität (Termonomer) zur Herstellung von vulkanisierbaren Ethylen-Propylen-Termonomer-Elastomeren (bekannt als EPDM).

Die Erfindung wird durch die folgenden Beispiele erläutert.

ppm bedeutet ppm, Masse/Masse, falls nicht anders angegeben.

Beispiele 1 und 2

Diskontinuierliche Herstellung von 5-Vinylnorbornen-(2)

In einen 3-1-Rührautoklaven (V4A-Stahl) wurden unter Stickstoff (10 ppm, Volumen/Volumen, Sauerstoff) je 500 g 1,3-Butadien und Dicyclopentadien (Molverhältnis 1,3-Butadien/Cyclopentadien = 1,2 : 1) und die in der Tabelle angegebenen Mengen NO gegeben. Der Reaktorinhalt wurde 5 h auf 176 °C erhitzt. Der Anfangsdruck bei 176 °C betrug 20 bar. Nach 5 h betrug der Druck ca. 10 bar. Nach dem Erkalten wurden der Reaktorinhalt und die Innenflächen des Reaktors beurteilt.

Tabelle

| Beispiel | NO /ppm/ | Beurteilung |
|---|---|---|
| 1 | 100 | klare Flüssigkeit, keine Ablagerungen |
| 2 | 1 000 | wie Beispiel 1 |

2

Beispiel 3

Kontinuierliche Herstellung von 5-Vinylnorbornen-(2)

In einem Rohrreaktor, dessen Volumen 1,2 l betrug, wurde die Luft durch Butan verdrängt. Dann wurde dem Reaktor ein Gemisch aus 1,3-Butadien, Dicyclopentadien (Massenverhältnis 1,5 : 1 ; Molverhältnis 1,3-Butadien/Cyclopentadien = 1,8 : 1) und 100 ppm NO mit einer Geschwindigkeit von 1,2 kg/h zugeführt. Dabei wurde das Gemisch in der Eingangszone des Reaktors auf 180 °C aufgeheizt und in der Reaktionszone auf dieser Temperatur gehalten. Der Druck im Reaktor betrug 12 bar.

Nach 300 h wurde der Versuch abgebrochen. Der Reaktoraustrag war während der gesamten Fahrperiode eine klare Flüssigkeit. Auf den Innenflächen des Reaktors konnten keine Ablagerungen festgestellt werden.

1,3-Butadien-Umsatz : 20,6 %

Dicyclopentadien-Umsatz : 65,5 %

Ausbeute an 5-Vinylnorbornen-(2) : 54 % der Theorie, bezogen auf das umgesetzte 1,3-Butadien. Die Ausbeute wurde durch destillative Trennung des Reaktoraustrags in drei Fraktionen und durch gaschromatografische Bestimmung des Vinylnorbornens und des nicht umgesetzten 1,3-Butadiens in den Fraktionen bestimmt.

**Patentansprüche**

1. Verfahren zum Verhindern der Bildung von polymeren Nebenprodukten bei der Herstellung von 5-Vinylnorbornen-(2) durch Diels-Alder-Reaktion von 1,3-Butadien (Dienkomponente) mit Cyclopentadien und/oder Dicyclopentadien (dienophile Komponente) ; das Verfahren weist die folgenden Merkmale auf : die Reaktionskomponenten und eine wirksame Menge eines Polymerisationsinhibitors werden unter (nahezu) vollständigem Ausschluß von Sauerstoff zusammengegeben ; das erhaltene Gemisch wird im Falle der Abwesenheit von Dicyclopentadien auf 140 bis 220 °C oder im Falle der Gegenwart von Dicyclopentadien auf 160 bis 220 °C erhitzt ; das Verfahren ist durch das folgende, zusätzliche Merkmal gekennzeichnet :

a) der Polymerisationsinhibitor ist Stickstoffmonoxid (NO) und/oder Distickstofftrioxid ($N_2O_3$).

2. Verfahren nach Anspruch 1, gekennzeichnet durch das folgende Merkmal :

b) der Polymerisationsinhibitor ist NO ; seine Menge ist 25 bis 1 000 ppm (Masse/Masse), bezogen auf die Summe der Dienkomponente und der dienophilen Komponente.

3. Verfahren nach Anspruch 2, gekennzeichnet durch das folgende Merkmal :

c) die Menge des Polymerisationsinhibitors ist 50 bis 500 ppm.

4. Verfahren nach einem der Ansprüche 1 bis 3, gekennzeichnet durch die folgenden Merkmale :

d) das Verfahren wird kontinuierlich durchgeführt ;

e) die Reaktionstemperatur ist 160 bis 210 °C ;

f) der Druck im Reaktor ist 5 bis 20 bar.

5. Verfahren nach Anspruch 4, gekennzeichnet durch die folgenden Merkmale :

g) das Verfahren wird in einem Rohrreaktor durchgeführt ;

h) die Reaktionstemperatur ist 170 bis 210 °C ;

i) der Druck ist 11 bis 15 bar.

6. Verfahren nach Anspruch 5, gekennzeichnet durch das folgende Merkmal :

j) der Druck ist 12 bis 14 bar.

**Claims**

1. A process for hindering the formation of polymeric by-products in the production of 5-vinylnorbornene-(2) by Diels-Alder reaction of 1,3-butadiene (diene component) with cyclopentadiene and/or dicyclopentadiene (dienophile component) ; the process has the following features : the reaction components and an effective amount of a polymerisation inhibitor are brought together under (almost) complete exclusion of oxygen ; the resulting mixture is heated to 140 to 220 °C if dicyclopentadiene is absent or to 160 to 220 °C if dicyclopentadiene is present ; the process is characterised by the following additional feature :

a) the polymerisation inhibitor is nitrogen monoxide (NO) and/or dinitrogen trioxide ($N_2O_3$).

2. A process according to claim 1, characterised by the following feature :

b) the polymerisation inhibitor is NO ; its amount is 25 to 1,000 ppm (mass/mass), based on the total of the diene component and the dienophile component.

3. A process according to claim 2, characterised by the following feature :

c) the amount of the polymerisation inhibitor is 50 to 500 ppm.

4. A process according to any of claims 1 to 3, characterised by the following features :

d) the process is carried out continuously ;

e) the reaction temperature is 160 to 210 °C ;

f) the pressure in the reactor is 5 to 20 bar.

5. A process according to claim 4, characterised by the following features :

g) the process is carried out in a tube reactor ;

h) the reaction temperature is 170 to 210 °C ;

i) the pressure is 11 to 15 bar.

6. A process according to claim 5, characterised by the following feature ;

j) the pressure is 12 to 14 bar.


**Revendications**

1. Procédé pour empêcher la formation de sous-produits polymères lors de la préparation de 5-vinylnorbornène-(2) par la réaction suivant Diels-Alder de 1,3-butadiène (composant diénique) sur du cyclo-pentadiène et/ou du dicyclo-pentadiène (composant diénophile) ; le procédé présente les caractéristiques suivantes : on place ensemble à l'abri (pratiquement) total d'oxygène les composants de la réaction et une quantité efficace d'un inhibiteur de polymérisation ; on chauffe le mélange obtenu, dans le cas de l'absence de dicyclo-pentadiène à une température de 140 à 220 °C ou, dans le cas de la présence de dicyclo-pentadiène, à une température de 160 à 220 °C ; le procédé est remarquable par la caractéristique complémentaire suivante :

a) l'inhibiteur de polymérisation est le monoxyde d'azote (NO) et/ou le trioxyde d'azote ($N_2O_3$).

2. Procédé selon la revendication 1, remarquable par la caractéristique suivante :

b) l'inhibiteur de polymérisation est NO ; sa quantité est de 25 à 1 000 ppm (masse/masse), relativement à la somme du composant diénique et du composant diénophile.

3. Procédé selon la revendication 2, remarquable par la caractéristique suivante :

c) la quantité de l'inhibiteur de polymérisation est de 50 à 500 ppm.

4. Procédé selon l'une des revendications 1 à 3, remarquable par les caractéristiques suivantes :

d) le procédé est mis en œuvre en continu ;

e) la température de réaction est de 160 à 210 °C ;

f) la pression dans le réacteur est de 5 à 20 bars.

5. Procédé selon la revendication 4, remarquable par les caractéristiques suivantes :

g) le procédé est mis en œuvre dans un réacteur tubulaire ;

h) la température de réaction est de 170 à 210 °C ;

i) la pression est de 11 à 15 bars.

6. Procédé selon la revendication 5, remarquable par la caractéristique suivante :

j) la pression est de 12 à 14 bars.